# EUROPEAN PATENT SPECIFICATION

(11) **EP 3 962 268 B1**
(45) Date of publication and mention of the grant of the patent: **23.07.2025**
(21) Application number: 20720439.7
(22) Date of filing: 20.04.2020
(51) Int. Cl.: A01N 43/58, A01N 25/02, A01N 25/10, A01P 7/04

(54) **STABILIZATION OF SUSPENSION CONCENTRATES BY HIGHLY SULFONATED LIGNOSULFONATE**
STABILISIERUNG VON SUSPENSIONSKONZENTRATEN DURCH HOCHSULFONIERTES LIGNINSULFONAT
STABILISATION DE CONCENTRÉS EN SUSPENSION AU MOYEN DE LIGNOSULFONATE HAUTEMENT SULFONÉ

(30) Priority: 03.05.2019 US 201962842670 P
(43) Date of publication of application: 09.03.2022
(73) Proprietor: BASF SE, 67056 Ludwigshafen am Rhein (DE)
(72) Inventor: XU, Wen, Research Triangle Park, NC 27709 (US); BENTON, Kara Walden, Research Triangle Park, NC 27709 (US)
(74) Representative: BASF IP Association
(86) International application number: PCT/EP2020/060984
(87) International publication number: WO 2020/224944

(56) References cited:
- EP-A1- 3 329 777
- WO-A1-2018/083040
- WO-A1-2020/224942
- WO-A1-2020/224943
- US-A1- 2018 184 654
- JERRY GARGULAK, STIG ARE GUNDERSEN, FRSDERIK BIERRE AND PAULINE ROLLAND: "Lignin-Based Chemicals as GreenDispersants for Liquid Formulation: Better Protection Against Crystal Growth", PESTICIDE FORMULATION AND DELIVERY SYSTEMS, vol. 36, 2016, pages 30 - 41, XP009521007

## Description

The invention relates to an aqueous agrochemical composition comprising suspended particles of dimpropyridaz that has a water-solubility of at least 1 g/l at 25 °C, and lignosulfonate. The invention also relates to a process for the preparation of the agrochemical composition of dimpropyridaz; to a method of controlling phytopathogenic fungi and/or undesired plant growth and/or undesired insect or mite attack and/or for regulating the growth of plants, wherein the agrochemical composition is allowed to act on the respective pests, their environment or the crop plants to be protected from the respective pest, on the soil and/or on undesired plants and/or on the crop plants and/or on their environment. It also relates to the agrochemical composition for use in a method of controlling or preventing infestation by insects or parasites of an animal. Another object is the use of the lignosulfonate for stabilizing an aqueous agrochemical composition comprising the suspended particles of the active ingredient; and a method of stabilizing an aqueous agrochemical composition comprising the suspended particles of the active ingredient comprising the contacting of the lignosulfonate with particles of the active ingredient and water. Combinations of embodiments with other embodiments, regardless of their respective level of preference, are within the scope of the invention.

Aqueous suspension concentrates are one of the most commonly used formulation types in the agrochemical industry. They are usually confined to active ingredients that have a very low water-solubility. Active ingredients with a high water-solubility are difficult to build into an aqueous suspension concentrates, since the particles either dissolve during storage or undergo Ostwald-Ripening, thereby resulting in increased particle size and eventually sedimentation. Other stability problems of such formulations are gelling, i.e. the formation of unstructured conglomerates of the active ingredient negatively influence the desired rheological profiles, e.g. by increasing the viscosity, or by clogging spray nozzles.

On the other hand, aqueous suspension concentrates have the advantage of a low content of organic solvents, and a relatively safe handling and application for the applicant.

It was therefore desirable to supply an aqueous agrochemical composition that contains an active ingredient with high water-solubility in the form of suspended particles, which is stable upon storage, does not undergo particle growth, particle aggregation or conglomeration, and thus is not prone to gelling or sedimentation of particles.

The objective has been achieved by an aqueous agrochemical composition comprising
a) suspended particles of dimpropyridaz and
b) lignosulfonate;
wherein the active ingredient has a water-solubility of at least 1 g/l at 25 °C.

The agrochemical composition is an aqueous agrochemical composition. The water content of the agrochemical composition is typically at least 1 wt%, preferably at least 5 wt%, more preferably at least 10 wt%, more preferably at least 15 wt%, most preferably at least 20 wt%, utmost preferably at least 25 wt%, especially preferably at least 30 wt%, and in particular at least 35 wt%, each time based on the total weight of the agrochemical compositions.

The water content of the agrochemical composition is typically up to 95 wt%, preferably up to 90 wt%, more preferably up to 80 wt%, most preferably up to 70 wt%, especially preferably up to 60 wt%, and in particular up to 50 wt% based on the total weight of the agrochemical composition. The water content of the agrochemical composition is typically of from 10 to 85 wt%, preferably from 10 to 65 wt%, more preferably from 15 to 60 wt% based on the total weight of the agrochemical composition.

The agrochemical composition comprises lignosulfonate, which is typically present in dissolved form. Lignosulfonate is commercially available, inter alia under the trade name Green-sperse S9 from Borregaard.

The lignosulfonate is preferably a sulfomethylated lignosulfonate. Such compounds fall under CAS number 68512-34-5.

The lignosulfonate typically has an organic sulfur content of at least 5 wt% based on the total weight of the lignosulfonate, preferably at least 6 wt%, more preferably at least 7 wt%, most preferably at least 8 wt%, utmost preferably at least 9 wt%. The lignosulfonate typically has an organic sulfur content of up to 15 wt% based on the total weight of the lignosulfonate, preferably up to 12 wt%, more preferably up to 11 wt%. The lignosulfonate typically has an organic sulfur content of from 4 to 12 wt% based on the total weight of the lignosulfonate, preferably from 5 to 10 wt%, more preferably from 7 to 10 wt%, most preferably from 8 to 10 wt%, and in particular from 8.5 to 9.5 wt%.

The term "organic sulfur content" refers to sulfur that is covalently bonded to the lignin scaffold. The covalent binding may be a direct bond to the lignin scaffold, or it may be indirectly boded over further carbon atoms, e.g. in sulfomethyl-groups.

The mass average molecular weight of the lignosulfonate is typically from 10 to 500 kDa, preferably 10 to 100 kDa, more preferably 10 to 60 kDa, and in particular 20 to 50 kDa. The mass average molecular weight of the lignosulfonate is typically up to 80 kDa, preferably up to 70 kDa, more preferably up to 55 kDa. The mass average molecular weight of the lignosulfonate is typically at least 5 kDa, preferably at least 15 kDa, more preferably at least 25 kDa.

The concentration of the lignosulfonate may be at least 0.1 wt%, preferably at least 0.2 wt%, more preferably at least 0.5 wt%, most preferably at least 1 wt%, and in particular at least 2 wt% based on the total weight of the agrochemical composition. In one embodiment, the concentration of the lignosulfonate is at least 2.5 wt% based on the total weight of the agrochemical composition. In another embodiment, the concentration of the lignosulfonate is at least 3.0 wt% based on the total weight of the agrochemical composition. The concentration of the lignosulfonate may be up to 20 wt%, preferably up to 15 wt%, more preferably up to 10 wt%, most preferably up to 8 wt%, and in particular up to 6 wt% based on the total weight of the agrochemical composition. The concentration of the lignosulfonate may be from 0.1 wt% to 15 wt%, preferably from 0.5 wt% to 10 wt%, more preferably from 1 to 5 wt% based on the total weight of the agrochemical composition.

The weight ratio of the active ingredient to the lignosulfonate is typically from 1:1 to 200:1, preferably from 1:1 to 50:1, more preferably from 2:1 to 50:1, most preferably from 3:1 to 40:1. Usually, the weight ratio of the active ingredient to the lignosulfonate is at least 5:1, preferably at least 6:1, more preferably at least 7:1.

The agrochemical composition contains dimpropyridaz as active ingredient.

The agrochemical composition comprises a pesticidally effective amount of the active ingredient. The term "effective amount" denotes an amount of the active ingredient, which is sufficient for controlling harmful fungi on cultivated plants or in the protection of materials and which does not result in a substantial damage to the treated plants. Such an amount can vary in a broad range and is dependent on various factors, such as the pest species to be controlled, the treated cultivated plant or material, the climatic conditions and the specific active ingredient used.

The concentration of the active ingredient in the agrochemical composition is typically at least 5 wt%, more preferably at least 10 wt%, most preferably at least 15 wt%, especially preferably at least 20 wt%, utmost preferably at least 25 wt%, and in particular at least 30 wt% based on the total weight of the agrochemical composition. The concentration of the active ingredient in the agrochemical composition is typically up to 95 wt%, preferably up to 85 wt%, more preferably up to 75 wt%, especially preferably up to 75 wt%, and in particular up to 65 wt% based on the total weight of the agrochemical composition. The agrochemical composition typically contains the active ingredient in a concentration of from 10 to 90 wt%, preferably of from 15 to 60 wt%, more preferably of from 20 to 50 wt% based on the total weight of the agrochemical composition.

The active ingredient is present in the agrochemical composition in the form of suspended particles. The particles may be characterized by their size distribution, which can be determined by dynamic light scattering techniques. Suitable dynamic light scattering measurement units are inter alia produced under the trade name Malvern Mastersizer 3000. The particles may be characterized by their median diameter, which is usually abbreviated as x50 value. The x50 value refers to a particular particle diameter, wherein half of the particle population by volume is smaller than this diameter. The x50 value is typically determined according to ISO 13320:2009.

The particles may have an x50 value of from 0.05 µm to 30 µm, preferably from 0.1 µm to 20 µm, more preferably from 0.5 to 20 µm, most preferably from 0.5 µm to15 µm, especially preferably from 0.5 µm to 10 µm. The particle typically have an x50 value of at least 0.75 µm, preferably at least 1 µm.

The active ingredient dimpropyridaz correspond to the name 1-(1,2-dimethylpropyl)-N-ethyl-5-methyl-N-pyridazin-4-yl-pyrazole-4-carboxamide.

The agrochemical composition may be prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005. The agrochemical composition is typically prepared by contacting the lignosulfonate with the active ingredient, preferably in the presence of water. In one embodiment, the resulting mixture is then subjected to milling or grinding to produce the suspension of the active ingredient. In another embodiment, the active ingredient is contacted with water and milled or grinded to produce an aqueous suspension of the active ingredient, whereupon said aqueous suspension is contacted with the lignosulfonate.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders. Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol. 1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids.

Suitable colorants (e.g. in red, blue, or green) are pigments of low water solubility and watersoluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

The agrochemical composition may optionally comprise further auxiliaries, such as 0,1-1 wt% bactericides, 5-15 wt% anti-freezing agents, 0,1-1 wt% anti-foaming agents, and 0,1-1 wt% colorants.

Solutions for seed treamtent (LS), Suspoemulsions (SE), flowable concentrates (FS), are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying the agrochemical composition on to plant propagation material, especially seeds include dressing, coating, pelleting, dusting, soaking and in-furrow application methods of the propagation material. Preferably, the agrochemical composition are applied on to the plant propagation material by a method such that germination is not induced, e. g. by seed dressing, pelleting, coating and dusting.

When employed in plant protection, the amounts of active ingredient applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active ingredient of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active ingredient applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the agrochemical composition as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

### Application methods

The agrochemical composition is suitable for use in protecting crops, plants, plant propagation materials, such as seeds, or soil or water, in which the plants are growing, from attack or infestation by animal pests. Therefore, the present invention also relates to a plant protection method, which comprises contacting crops, plants, plant propagation materials, such as seeds, or soil or water, in which the plants are growing, to be protected from attack or infestation by animal pests, with a pesticidally effective amount of the agrochemical composition.

The agrochemical composition suitable for use in combating or controlling animal pests. Therefore, the present invention also relates to a method of combating or controlling animal pests, which comprises contacting the animal pests, their habitat, breeding ground, or food supply, or the crops, plants, plant propagation materials, such as seeds, or soil, or the area, material or environment in which the animal pests are growing or may grow, with a pesticidally effective amount of the agrochemical composition of the present invention.

The agrochemical composition is effective through both contact and ingestion. Furthermore, the agrochemical composition can be applied to any and all developmental stages, such as egg, larva, pupa, and adult.

The application can be carried out both before and after the infestation of the crops, plants, plant propagation materials, such as seeds, soil, or the area, material or environment by the pests.

Suitable application methods include inter alia soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the agrochemical composition to the furrow, and closing the furrow. Foliar application refers to the application of the agrochemical composition to plant foliage, e.g. through spray equipment. For foliar applications, it can be advantageous to modify the behavior of the pests by use of pheromones in combination with agrochemical composition. Suitable pheromones for specific crops and pests are known to a skilled person and publicly available from databases of pheromones and semiochemicals, such as http://www.pherobase.com.

As used herein, the term "contacting" includes both direct contact (applying the agrochemical composition directly on the animal pest or plant - typically to the foliage, stem or roots of the plant) and indirect contact (applying the agrochemical composition to the locus, i.e. habitat, breeding ground, plant, seed, soil, area, material or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet or fodder beet; fruits, such as pomes, stone fruits or soft fruits, e.g. apples, pears, plums, peaches, nectarines, almonds, cherries, papayas, strawberries, raspberries, blackberries or gooseberries; leguminous plants, such as beans, lentils, peas, alfalfa or soybeans; oil plants, such as rapeseed (oilseed rape), turnip rape, mustard, olives, sunflowers, coconut, cocoa beans, castor oil plants, oil palms, ground nuts or soybeans; cucurbits, such as squashes, pumpkins, cucumber or melons; fiber plants, such as cotton, flax, hemp or jute; citrus fruit, such as oranges, lemons, grapefruits or mandarins; vegetables, such as eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, such as avocados, cinnamon or camphor; energy and raw material plants, such as corn, soybean, rapeseed, sugar cane or oil palm; tobacco; nuts, e.g. walnuts; pistachios; coffee; tea; bananas; vines (table grapes and grape juice grape vines); hop; sweet leaf (also called Stevia); natural rubber plants or ornamental and forestry plants, such as flowers (e.g. carnation, petunias, geranium/pelargoniums, pansies and impatiens), shrubs, broad-leaved trees (e.g. poplar) or evergreens, e.g. conifers; eucalyptus; turf; lawn; grass such as grass for animal feed or ornamental uses. Preferred plants include potatoes sugar beets, tobacco, wheat, rye, barley, oats, rice, corn, cotton, soybeans, rapeseed, legumes, sunflowers, coffee or sugar cane; fruits; vines; ornamentals; or vegetables, such as cucumbers, tomatoes, beans or squashes.

The term "cultivated plants" is to be understood as including plants which have been modified by mutagenesis or genetic engineering in order to provide a new trait to a plant or to modify an already present trait.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

Advantages: the agrochemical composition is characterized by a very high storage stability, reduced particle growth (e.g. crystal growth), reduced sedimentation, reduced gelling, an advantageous rheological profile, and high biological efficacy. Hence, the term "stability" as used herein typically refers to physical stability of the agrochemical formulation. Accordingly, the term "stabilizing" usually refers to a physical stabilization (e.g. increase of storage stability).

The following examples illustrate the invention.

### Examples

The following ingredients were used for preparing the agrochemical compositions of the examples.

Insecticide A: 1-[(1RS)-1,2-dimethylpropyl]-N-ethyl-5-methyl-N-pyridazin-4-yl-1H-pyrazole-4-carboxamide.

Polymeric Stabilizer A: sulfomethylated lignosulfonate, organic sulfur content approximately 9 wt%, mass average molecular weight 20 to 50 kDa

Polymeric Stabilizer B: polyoxypropylene-polyoxyethylene block copolymer, liquid at 20 °C, dynamic viscosity 750 mPas at 25 °C, average molecular weight 6500

Polymeric Staiblizer C: nonionic ethoxylated and propoxylated butyl alcohol, HLB 17, solid at 25 °C

Polymeric Stabilizer D: reaction product of methyl methacrylate, methacrylic acid and methoxy polyethylene glycol methacrylate, dynamic viscosity at 25 °C 800 to 2000 mPas Wetting Agent: mixture of sulfated alkylcarboxylate with sulfonated alkylnaphthalene sodium salt Antifoam: emulsion of dimethylsiloxan on silica particles, defoamer content 20 wt%

Biocide A: glycol based solution of benzisothiazolinone

Biocide B: aqueous composition of benzisothiazolinones and 5-chloro-2-methylisothiazolin-3-one

Thickener: magnesium aluminium silicate, smectite

Additive A: hydrophobic fumed silica, surface modified with dimethyldichlorosilane, surface area 130 m²/g

### Example-1

Suspension concentrate SC-1 and comparative suspension concentrates SC-C1, SC-C2, and SC-C3 were prepared containing the ingredients according to Table A. The suspension concentrates differed by the type of Polymeric Stabilizer used, as provided in Table B.

The suspension concentrates were prepared by adding in a first step Insecticide A, Wetting Agent, Biocide A, Biocide B, Thickener, Acetic Acid, Antifoam, and Additive A to water.

The resulting mill base was homogenized in a second step until uniform, and then milled in a bead mill until a mean particle size of 2-3 microns was achieved.

**Table A: Ingredients of suspension concentrates SC-1, SC-C1, SC-C2, and SC-C3**

| **Component** | **Concentration [wt%]** |
|---|---|
| Insecticide A | 31.88 |
| Wetting Agent | 4.50 |
| Polymeric Stabilizer | 3.00 |
| Antifoam | 0.40 |
| Biocide A | 0.20 |
| Biocide B | 0.10 |
| Thickener | 0.25 |
| Acetic acid | 0.22 |
| Additive A | 1.00 |
| Water | to 100 |

**Table B: Polymeric Thickeners contained in suspension concentrates SC-1, SC-C1, SC-C2, SC-C3; and results of stability analysis after incubation in the cycling chamber.**

| Suspension Concentrate | Polymeric Stabilizer | Stability Analyses after incubation in the cycling chamber | |
|---|---|---|---|
| | | Incubation two weeks 20 °C / 40 °C cycling | Incubation four weeks -10 °C / 30 °C cycling |
| SC-1 | Polymeric Thickener A | Flowable | No crystal growth (less than 7 µm) |
| SC-C1 | Polymeric Thickener B | Gelled | Significant crystal growth (up to 23 µm) |
| SC-C2 | Polymeric Thickener C | Gelled | Significant crystal growth (up to 16 µm) |
| SC-C3 | Polymeric Thickener D | Gelled | Significant crystal growth (up to 27 µm) |

The suspension concentrates were then placed in either a 20 °C / 40 °C cycling chamber (24 hours at 20 °C and 24 hours at 40 °C) for two weeks, or placed in a -10 °C / 30 °C cycling chamber (24 hours at -10 °C and 24 hours at 40 °C) for four weeks, upon which the suspension concentrates were analyzed by visual inspection and testing of its rheological profile, and by measuring the crystal growth of particles under a microscope. The results were summarized in Table B.

### Example-2

Suspension concentrates SC-2 to SC-6, and comparative suspension concentrate SC-C4, were prepared containing the ingredients according to Table C. The suspension concentrates differed by the amount of Polymeric Stabilizer A used, as provided in Table D.

The suspension concentrates were prepared by adding in a first step Insecticide A, Wetting Agent, Biocide A, Biocide B, Thickener, Acetic Acid, Antifoam, and Additive A to water.

The resulting mill base was homogenized in a second step until uniform, and then milled in a bead mill until a mean particle size of 2-3 microns was achieved.

The suspension concentrates were then placed in either a 20 °C / 40 °C cycling chamber (24 hours at 20 °C and 24 hours at 40 °C) for two weeks, or placed in a -10 °C / 30 °C cycling chamber (24 hours at -10 °C and 24 hours at 40 °C) for four weeks, upon which the suspension concentrates were analyzed by visual inspection and testing of its rheological profile, and by measuring the crystal growth of particles under a microscope. The results were summarized in Table D.

**Table C: Ingredients of suspension concentrates SC-2, to SC-6, and SC-C4.**

| **Component** | **Concentration [wt%]** |
|---|---|
| Insecticide A | 31.88 |
| Wetting Agent | 4.50 |
| Polymeric Stabilizer A | see Table D |
| Antifoam | 0.40 |
| Biocide A | 0.20 |
| Biocide B | 0.10 |
| Thickener | 0.25 |
| Acetic acid | 0.22 |
| Additive A | 1.00 |
| Water | to 100 |

**Table D: Concentration of Polymeric Stabilizer A in SC-C4, and SC-2 to SC-4, as well as observations on rheological properties and crystal growth under different cycling conditions.**

| Suspension Concentrate | Concentration of Polymeric Stabilizer A in wt% | Stability Analyses after incubation for three weeks in the -10 °C / 30 °C cycling chamber | Stability Analyses after incubation for two weeks in the 20 °C / 40 °C cycling chamber |
|---|---|---|---|
| SC-C4 | 0.0 | Very significant crystal growht | Gelled |
| SC-2 | 1.0 | Significant crystal growth | Semi-flowable |
| SC-3 | 2.0 | Crystal growth detected | Very flowable |
| SC-4 | 3.0 | Minimal crystal growth | Very flowable |
| SC-5 | 4.0 | Minimal crystal growth | Very flowable |
| SC-6 | 5.0 | No crystal growth | Very flowable |

## Claims

1. Aqueous agrochemical composition comprising
a) suspended particles of dimpropyridaz; and
b) lignosulfonate.

2. Agrochemical composition of claim 1, wherein the lignosulfonate has an organic sulfur content of at least 5 wt% based on the total weight of the lignosulfonate.

3. Agrochemical composition of claim 1 or 2, wherein the lignosulfonate has an organic sulfur content of at least 8 wt% based on the total weight of the lignosulfonate.

4. Agrochemical composition according to any of claims 1 to 3, containing the lignosulfonate in a concentration of from 0.5 to 10 wt% based on the total weight of the agrochemical composition.

5. Agrochemical composition according to any of claims 1 to 4, containing the lignosulfonate in a concentration of at least 1 wt% based on the total weight of the agrochemical composition.

6. The agrochemical composition according to any of claims 1 to 5, wherein the weight ratio of dimpropyridaz to the lignosulfonate is from 1:1 to 50:1.

7. The agrochemical composition according to any of claims 1 to 6, wherein the suspended particles of dimpropyridaz have a mean diameter of from 0.5 µm to 20 µm.

8. The agrochemical composition according to any of claims 1 to 7, wherein the concentration of the suspended particles of dimpropyridaz in the agrochemical composition is at least 5 wt%.

9. The agrochemical composition according to any of claims 1 to 8, containing a second active ingredient selected from fungicides, insecticides, and herbicides.

10. Use of the lignosulfonate as defined in any of claims 1 to 6 for stabilizing an aqueous agrochemical composition comprising suspended particles of dimpropyridaz as defined in any of claims 1 to 9.

11. Method of stabilizing an aqueous agrochemical composition comprising suspended particles of dimpropyridaz as defined in any of claims 1 to 9, comprising the step of contacting lignosulfonate as defined in any of claims 1 to 6 with particles of dimpropyridaz as defined in any of claims 1 to 9 and water.

12. Composition according to any of claims 1 to 9 for use in a method of controlling or preventing infestation by insects or parasites of an animal.

13. Method for the preparation of the agrochemical composition as defined in any of claims 1 to 9 comprising the step of contacting the lignosulfonate as defined in any of claims 1 to 6 with dimpropyridaz as defined in any of claims 1 to 9 in the presence of water.

14. Plant propagation material comprising the agrochemical composition as defined in any of claims 1 to 9.

15. Non-therapeutic Use of the agrochemical composition according to any of claims 1 to 9 for combating or controlling animal pests.

## Patentansprüche

1. Wässrige agrochemische Zusammensetzung, umfassend
a) suspendierte Partikel von Dimpropyridaz; und
b) Lignosulfonat.

2. Agrochemische Zusammensetzung nach Anspruch 1, wobei das Lignosulfonat einen organischen Schwefelgehalt von mindestens 5 Gew.-%, bezogen auf das Gesamtgewicht des Lignosulfonats, aufweist.

3. Agrochemische Zusammensetzung nach Anspruch 1 oder 2, wobei das Lignosulfonat einen organischen Schwefelgehalt von mindestens 8 Gew.-%, bezogen auf das Gesamtgewicht des Lignosulfonats, aufweist.

4. Agrochemische Zusammensetzung nach einem der Ansprüche 1 bis 3, die das Lignosulfonat in einer Konzentration von 0,5 bis 10 Gew.-%, bezogen auf das Gesamtgewicht der agrochemischen Zusammensetzung, enthält.

5. Agrochemische Zusammensetzung nach einem der Ansprüche 1 bis 4, die das Lignosulfonat in einer Konzentration von mindestens 1 Gew.-%, bezogen auf das Gesamtgewicht der agrochemischen Zusammensetzung, enthält.

6. Agrochemische Zusammensetzung nach einem der Ansprüche 1 bis 5, wobei das Gewichtsverhältnis von Dimpropyridaz zu Lignosulfonat 1:1 bis 50:1 beträgt.

7. Agrochemische Zusammensetzung nach einem der Ansprüche 1 bis 6, wobei die suspendierten Dimpropyridaz-Partikel einen mittleren Durchmesser von 0,5 µm bis 20 µm aufweisen.

8. Agrochemische Zusammensetzung nach einem der Ansprüche 1 bis 7, wobei die Konzentration der suspendierten Dimpropyridaz-Partikel in der agrochemischen Zusammensetzung mindestens 5 Gew.-% beträgt.

9. Agrochemische Zusammensetzung nach einem der Ansprüche 1 bis 8, die einen zweiten Wirkstoff enthält, der aus Fungiziden, Insektiziden und Herbiziden ausgewählt ist.

10. Verwendung des Lignosulfonats gemäß einem der Ansprüche 1 bis 6 zur Stabilisierung einer wässrigen agrochemischen Zusammensetzung, die suspendierte Dimpropyridaz-Partikel gemäß einem der Ansprüche 1 bis 9 enthält.

11. Verfahren zum Stabilisieren einer wässrigen agrochemischen Zusammensetzung, die suspendierte Dimpropyridaz-Partikel gemäß der Definition in einem der Ansprüche 1 bis 9 enthält, umfassend den Schritt des Inkontaktbringens von Lignosulfonat gemäß der Definition in einem der Ansprüche 1 bis 6 mit Dimpropyridaz-Partikeln gemäß der Definition in einem der Ansprüche 1 bis 9 und Wasser.

12. Zusammensetzung gemäß einem der Ansprüche 1 bis 9 zur Verwendung in einem Verfahren zur Bekämpfung oder Verhinderung des Befalls eines Tieres durch Insekten oder Parasiten.

13. Verfahren zur Herstellung der agrochemischen Zusammensetzung gemäß einem der Ansprüche 1 bis 9, umfassend den Schritt des Inkontaktbringens des Lignosulfonats gemäß einem der Ansprüche 1 bis 6 mit Dimpropyridaz gemäß einem der Ansprüche 1 bis 9 in Gegenwart von Wasser.

14. Pflanzenvermehrungsmaterial, das die agrochemische Zusammensetzung gemäß einem der Ansprüche 1 bis 9 umfasst.

15. Nicht-therapeutische Verwendung der agrochemischen Zusammensetzung gemäß einem der Ansprüche 1 bis 9 zur Bekämpfung oder Kontrolle von tierischen Schädlingen.

## Revendications

1. Composition agrochimique aqueuse comprenant
a) particules de dimpropyridaz en suspension; et
b) lignosulfonate.

2. Composition agrochimique selon la revendication 1, dans laquelle le lignosulfonate a une teneur en soufre organique d'au moins 5 % en poids par rapport au poids total du lignosulfonate.

3. Composition agrochimique selon la revendication 1 ou 2, dans laquelle le lignosulfonate a une teneur en soufre organique d'au moins 8 % en poids par rapport au poids total du lignosulfonate.

4. Composition agrochimique selon l'une quelconque des revendications 1 à 3, contenant le lignosulfonate dans une concentration de 0,5 à 10 % en poids par rapport au poids total de la composition agrochimique.

5. Composition agrochimique selon l'une quelconque des revendications 1 à 4, contenant le lignosulfonate dans une concentration d'au moins 1 % en poids par rapport au poids total de la composition agrochimique.

6. Composition agrochimique selon l'une quelconque des revendications 1 à 5, dans laquelle le rapport pondéral du dimpropyridaz au lignosulfonate est de 1:1 à 50:1.

7. Composition agrochimique selon l'une quelconque des revendications 1 à 6, dans laquelle les particules en suspension de dimpropyridaz ont un diamètre moyen de 0,5 pm à 20 pm.

8. Composition agrochimique selon l'une quelconque des revendications 1 à 7, dans laquelle la concentration des particules en suspension de dimpropyridaz dans la composition agrochimique est d'au moins 5 % en poids.

9. Composition agrochimique selon l'une quelconque des revendications 1 à 8, contenant un second ingrédient actif choisi parmi les fongicides, les insecticides et les herbicides.

10. Utilisation du lignosulfonate tel que défini dans l'une quelconque des revendications 1 à 6 pour stabiliser une composition agrochimique aqueuse comprenant des particules en suspension de dimpropyridaz telles que définies dans l'une quelconque des revendications 1 à 9.

11. Procédé de stabilisation d'une composition agrochimique aqueuse comprenant des particules en suspension de dimpropyridaz telles que définies dans l'une quelconque des revendications 1 à 9, comprenant l'étape de mise en contact de lignosulfonate tel que défini dans l'une quelconque des revendications 1 à 6 avec des particules de dimpropyridaz telles que définies dans l'une quelconque des revendications 1 à 9 et de l'eau.

12. Composition selon l'une quelconque des revendications 1 à 9, destinée à être utilisée dans un procédé de contrôle ou de prévention de l'infestation par des insectes ou des parasites d'un animal.

13. Procédé de préparation de la composition agrochimique telle que définie dans l'une quelconque des revendications 1 à 9 comprenant l'étape de mise en contact du lignosulfonate tel que défini dans l'une quelconque des revendications 1 à 6 avec du dimpropyridaz tel que défini dans l'une quelconque des revendications 1 à 9 en présence d'eau.

14. Matériel de propagation végétale comprenant la composition agrochimique telle que définie dans l'une quelconque des revendications 1 à 9.

15. Utilisation non thérapeutique de la composition agrochimique selon l'une quelconque des revendications 1 à 9 pour lutter contre ou contrôler les animaux nuisibles.
